# EUROPEAN PATENT APPLICATION

(11) **EP 1 859 807 A1**
(43) Date of publication of application: **28.11.2007**
(21) Application number: 06715000.3
(22) Date of filing: 01.03.2006
(51) Int. Cl.: A61K 45/00, A61K 31/138, A61K 31/4525, A61P 25/18, A61P 25/30

(54) **RECURRENCE PREVENTIVE THERAPEUTIC AGENT FOR PSYCHOSTIMULANT-INDUCED PSYCHOSIS AND SCHIZOPHRENIA**

(30) Priority: 04.03.2005 JP 2005061635; 20.06.2005 JP 2005179564
(71) Applicant: Tokyo Medical and Dental University, Tokyo 113-8510 (JP)
(72) Inventor: KASHIWA, Atsushi, hima 1-chome, Bunkyo-ku, Tokyo, 1138510 (JP); NISHIKAWA, Toru, hima 1-chome, Bunkyo-ku, Tokyo, 1138510 (JP); KANEKO, Yujiro, hima 1-chome, Bunkyo-ku, Tokyo, 1138510 (JP)
(74) Representative: Hart Davis, Jason
(86) International application number: PCT/JP2006/303883
(87) International publication number: WO 2006/093192

(57) **Abstract**

The present invention provides a therapeutic agent for preventing recurrence of psychostimulant-induced psychosis and schizophrenia. This therapeutic agent for preventing recurrence of psychostimulant-induced psychosis and schizophrenia includes a drug that amplifies serotonin signals as an active component. This therapeutic agent for preventing recurrence of psychostimulant-induced psychosis and schizophrenia includes a drug that amplifies serotonin signals as an active component. This therapeutic agent for preventing recurrence of psychostimulant-induced psychosis and schizophrenia includes a drug that amplifies serotonin signals as an active component.

## Description

### TECHNICAL FIELD

The present invention relates to a therapeutic agent for preventing recurrence of psychostimulant-induced psychosis and schizophrenia including a drug that amplifies serotonin signals as an active component.

### BACKGROUND ART

Psychostimulant-induced psychosis and schizophrenia have a lot in common. Of course, the psychostimulant-induced psychosis is developed due to habitual use of a psychostimulant, and hallucination delusion state is induced as an acute symptom, and the image of the states closely resembles schizophrenia. A treatment for the hallucination delusion state has been established, in which a dopamine D2 antagonist called "antipsychotic agent" is used. On the other hand, there however is a problem that such diseases are readily recurrent, so that it can be often observed that the diseases recur and induce the hallucination delusion state even after the acute phase treatment ends.

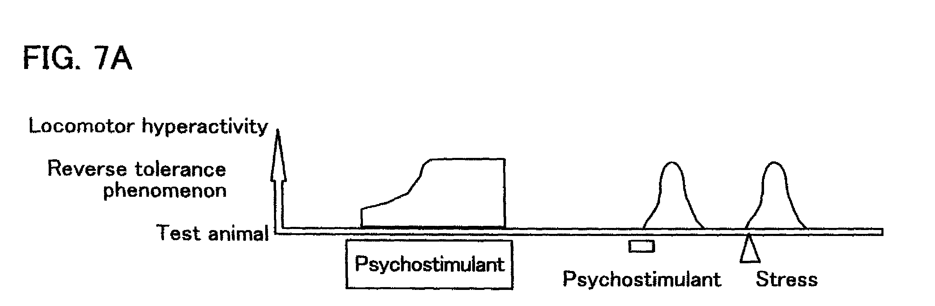

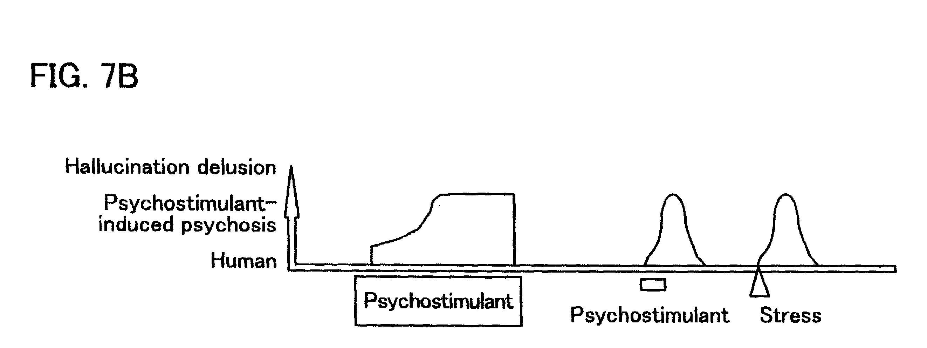

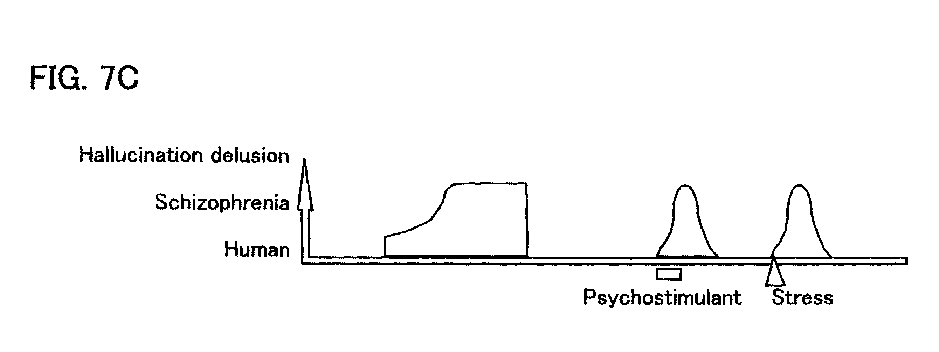

FIG. 7 shows phenomena of reverse tolerance and schizophrenia. "Reverse tolerance phenomenon" referred to herein means a phenomenon that by repeatedly administrating a psychostimulant such as amphetamine or methamphetamine, cocaine, or methylphenidate to an animal, its sensitivity against such a drug is increased to lower the threshold, whereby the abnormal behavior is more likely to be developed. FIG. 7A shows the reverse tolerance phenomenon, the vertical axis shows the alteration of the activity quantity in the test animal. When a psychostimulant is administered to an animal, it starts to move around. This behavior is referred to as a locomotor hyperactivity. The response to psychostimulants can be determined by measuring the locomotor hyperactivity. Thus, once reverse tolerance is formed by administrating psychostimulants, abnormal behavior occurs, even though a small amount of psychostimulant is administrated, not normally producing abnormal behavior. It is known that this phenomenon is maintained semipermanently and the similar increased sensitivity to stresses can be observed, once it is formed. As shown in FIG. 7B, the reverse tolerance phenomenon to psychostimulants occurs also in a human. In the Figure, the vertical axis shows the extent of the hallucination delusion. Reverse tolerance is formed in a human like an animal by repeatedly administrating psychostimulants, so that when a small amount of psychostimulant generally not causing the hallucination delusion state is used again, such a state is caused even if psychostimulants are not used for a long period afterwards. In addition, even various kinds of stresses including an emotional stress cause a hallucination delusion state, which is also referred to as "flashback". FIG. 7C shows the state in connection with schizophrenia. Thus, schizophrenia is developed as a first episode, and then the patient becomes sensitive to various kinds of stresses which may trigger developing schizophrenia. Also, a patient with schizophrenia is hypersensitive to psychostimulants. There is a report in the U.S. that the hallucination delusion state is also caused even when a small amount of the psychostimulant is administered. What is common in psychostimulant-induced psychosis and schizophrenia is that this readily recurrent state is maintained semipermanently, which makes it difficult to cure radically these diseases.

Regarding this reverse tolerance phenomenon, it can be considered two phases separately: formation of reverse tolerance by repeatedly administrating psychostimulants, and maintenance of reverse tolerance that the hallucination delusion state recurs by using a small amount of the psychostimulant. For the formation of reverse tolerance, it is known that formation of the reverse tolerance is avoided by the dopamine D2 antagonist generally used for these disease as an antipsychotic agent. However, the dopamine D2 antagonist insufficiently affects reverse tolerance which had been formed once, so that it cannot completely prevent recurrence of the diseases. A patient who may be accompanied by a clinical problem in fact has the reverse tolerance already formed by administrating psychostimulants, so that it is important to allow the maintained reverse tolerance to disappear. If it is possible to allow the maintained reverse tolerance to semipermanently disappear, prognoses can be improved.

Among past reports, some reported disappearance of problematic maintenance of the reverse tolerance. In addition, it is also reported that maintenance of the reverse tolerance disappears by administrating a D1 agonist (Non-patent document 1), or a combination of an NMDA antagonist and a D2 agonist (Non-patent document 1), or coadministrating cocaine and a serotonin 2A antagonist (Non-patent document 2).

Non-patent document 1: Li Y., White F.J., Wolf M.E. (2000) Pharmacological reversal of behavioral and cellular indices of cocaine sensitization in the rat. Psychopharmacology 151, 175_183.

Non-patent document 2: Davidson C., Lazarus C., Xoing X., Lee T.H., Ellinwood E.H. (2002) 5-HT2 receptor antagonists given in the acute withdrawal from daily cocaine injections can reverse established sensitization. European Journal of Pharmacal. 453, 255_263.

### DISCLOSURE OF THE INVENTION

### Problem to be Solved by the Invention

However, both methods described in Non-patent document 1 and 2 cannot be clinically used.

The present invention was made in view of the abovementioned problems, and an object thereof is to provide a therapeutic agent for preventing recurrence of psychostimulant-induced psychosis and schizophrenia.

### Means for Solving the Problem

As a result that the inventors have studied elaborately in order to solve the problem, they have found that reverse tolerance disappears under a condition of an excessive amount of serotonin even if reverse tolerance had been formed once. Accordingly, the present invention was accomplished. More specifically, the present invention provides as follows.

According to a first aspect of the present invention, there is provided a therapeutic agent for preventing recurrence of psychostimulant-induced psychosis and schizophrenia including a drug that amplifies synaptic serotonin signals as an active component.

According to the therapeutic agent for preventing recurrence of psychostimulant-induced psychosis and schizophrenia, a drug that amplifies serotonin signals is included as an active component, so that a state in which synaptic serotonin is present in an excessive amount can be provided by administering the agent to allow reverse tolerance to disappear.

According to a second aspect of the present invention, there is provided the therapeutic agent for preventing recurrence of psychostimulant-induced psychosis and schizophrenia according to the first aspect, wherein the drug that amplifies synaptic serotonin signals is a selective serotonin reuptake inhibitor (SSRI) or a double serotonin-noradrenaline reuptake inhibitor (SNRI).

According to this aspect, SSRI or SNRI binds to a transporter that inhibits reuptake of serotonin into a synaptic cleft to inhibit reuptake of serotonin. Thus, serotonin in the synaptic cleft is increased to enable disappearance of reverse tolerance, so that this aspect is effective as a therapeutic agent for preventing recurrence of a psychostimulant-induced psychosis and the schizophrenia.

According to a third aspect of the present invention, there is provided the therapeutic agent for preventing recurrence of psychostimulant-induced psychosis and schizophrenia according to the first or second aspect of the present invention, wherein the drug that amplifies serotonin signals is fluoxetine (C₁₇H₁₈F₃NO) represented by the following formula (1), paroxetine (C₁₉H₂₀FNO₃) represented by the following formula (2), and/or derivatives thereof.

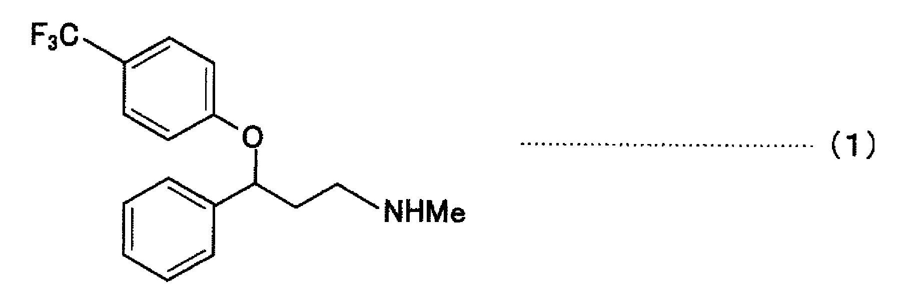

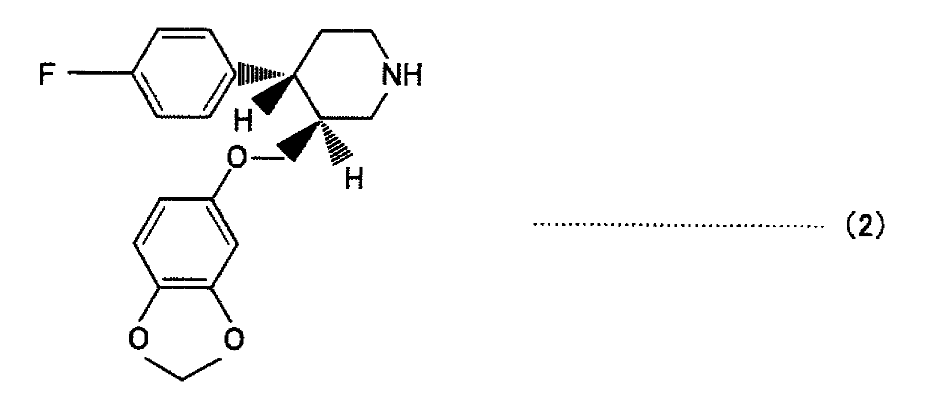

According to this aspect, the drug that amplifies serotonin signals is fluoxetine and/or paroxetine, so that this aspect is effective as a therapeutic agent for preventing recurrence of psychostimulant-induced psychosis and schizophrenia, particularly in terms of the effect and side effect.

### Effect of the Invention

According to the present invention, a therapeutic agent for preventing recurrence of psychostimulant-induced psychosis and schizophrenia can be provided.

### BRIEF DESCRIPTION OF THE DRAWINGS

FIG. 1 shows a schematic view illustrating a test procedure for formation/maintenance of reverse tolerance.
FIG. 2 shows a graph illustrating formation/maintenance of reverse tolerance by MAP.
FIG. 3 shows a schematic view illustrating a schedule of fluoxetine administration to a MAP reverse tolerance model.
FIG. 4 shows a graph illustrating disappearance of reverse tolerance by fluoxetine.
FIG. 5 shows a schematic view illustrating a schedule of paroxetine administration to an MAP reverse tolerance model.
FIG. 6 shows a graph illustrating disappearance of reverse tolerance by paroxetine.
FIG. 7 shows a schematic chart illustrating a reverse tolerance phenomenon.

### PREFERRED MODE FOR CARRYING OUT THE INVENTION

The therapeutic agent for preventing recurrence of psychostimulant-induced psychosis and schizophrenia of the present invention is not limited particularly as long as a drug that amplifies serotonin signals is included as an active component. In particular, the therapeutic agent is preferably produced to include a selective serotonin reuptake inhibitor (SSRI) or a double serotonin-noradrenaline reuptake inhibitor (SNRI) as the main component.

SSRI used in the present invention is not particularly limited ; however for example, fluoxetine, sertraline, paroxetine, fluvoxamine, citalopram, escitalopram, and/or a derivative thereof can be used. In addition, SNRI is not limited in particular; however milnacipran, duloxetine, venlafaxine and/or a derivative thereof can be used. In addition, these SSRIs or SNRIs may be used alone or in combination of two kinds or more.

### Dosage forms

The therapeutic agent for preventing recurrence of psychostimulant-induced psychosis and schizophrenia of the present invention can be combined with a required additive in addition to SSRI and SNRI to prepare as a solid oral formulation, a liquid oral formulation, or a parenteral formulation such as an injectable solution according to a conventional method. The solid oral formulation is most preferable.

For preparation of the solid oral formulation, an excipient such as lactose, mannitol, glucose, microcrystalline cellulose, starch, or corn starch may be added to thereafter produce a tablet, a granule, a powder, a capsule, or the like. In addition, the solid oral formulation can be prepared by admixing with, in addition to the excipient, a binder such as hydroxypropylcellulose or hydroxypropyl methylcellulose (HPMC), a lubricant such as magnesium stearate, polyethylene glycol, starch or talc, a disintegrator such as calcium carboxymethylcellulose or carmellose calcium, a stabilizer such as lactose, a solubilizer such as glutaminic acid or aspartic acid, a plasticizer such as polyethylene glycol, and a colorant such as titanium oxide, talc or yellow iron oxide, if necessary. Furthermore, the tablet or the pill may be coated with glycocalyx such as saccharose, gelatin, agar, pectin, hydroxypropylcellulose, and hydroxypropylmethylcellulose phthalate or a film of a gastric-or enteric-coating material as required.

For preparation of the liquid oral formulation, an inert diluent such as purified water or ethanol, a flavor modifier, a buffer, a stabilizer, a fragrance modifier and the like may be added to produce a liquid agent for internal use, a syrup, a gel, a elixir, and the like.

For the injectable solution, a sterile aqueous or non-aqueous solution, a suspension, an emulsion, or the like may be prepared to produce subcutaneous, intramuscular and intravenous injectable solutions. The diluent of the aqueous solution and suspension can include distilled water and saline for injection. In addition, the diluent of the non-aqueous solution and suspension can include propylene glycol, polyethylene glycol, vegetable oil such as olive oil, alcohols such as ethanol. Furthermore, a pH adjustor, a buffer, a preservative, a humectant, an emulsifier, a dispersing agent, a stabilizer (e.g., lactose), an isotonizing agent, a local anesthetic, a solubilizer (e. g., glutaminic acid, aspartic acid) or the like may be added if necessary.

### Effective dose

The effective dose of the therapeutic agent for preventing recurrence of psychostimulant-induced psychosis and schizophrenia may vary depending on the weight, age, and sex of patients, the administration method, physical condition, symptoms, dosage form, and the like. However, in the case of oral administration to adults, 20 mg to 60 mg of fluoxetine or 10 mg to 40 mg of paroxetine are preferably taken in divided one or two to several doses respectively per day.

### EXAMPLES

The present invention is explained in detail by way of Examples below but not limited thereto.

### Test for formation/maintenance of reverse tolerance

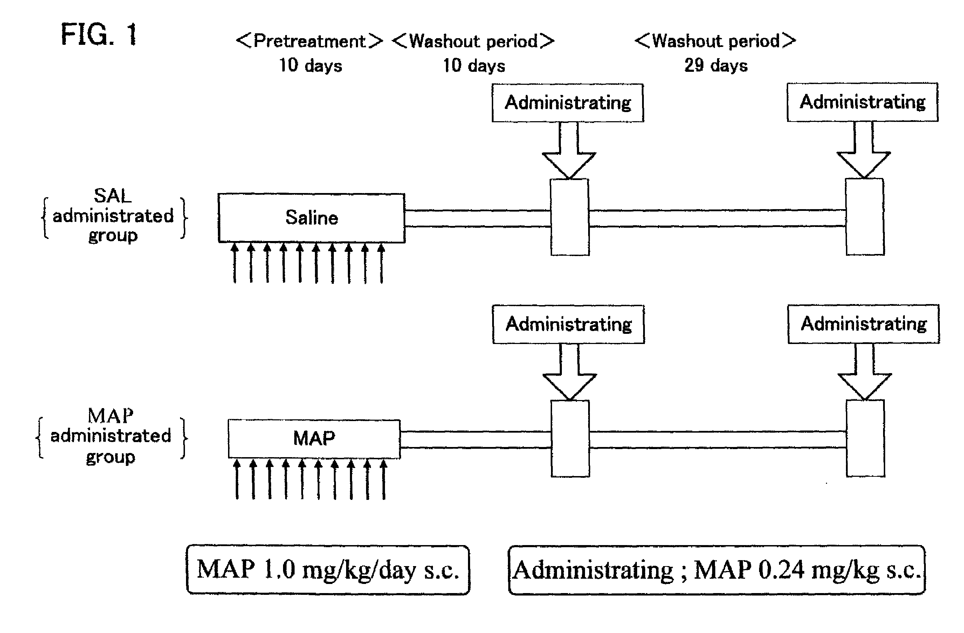

Methamphetamine (hereinafter, may be also referred to as "MAP") which is a psychostimulant was administered to an animal, and then formation/maintenance of reverse tolerance was verified. In this test, eight-week-old male ddy mice were used. FIG. 1 shows the test procedure. First, mice were divided into two groups, and then 1 mg/kg/day MAP and saline were respectively administered to each group for ten days as pretreatment. The agent was prepared by dissolving in saline and administered by subcutaneous injection. Hereinafter, the group to which MAP was administered is called "MAP administrated group", and the group to which saline was administered is called "SAL administrated group". MAP of 1 mg/kg used here corresponds an amount to result in increase in locomotor hyperactivity when it is administered to mice. In addition, a first administration of MAP was conducted after a 10-day washout period in order to confirm if this phenomenon was maintained. The dosage, 0.24 mg/kg, was less than that of the pretreatment, which is reportedly a quantity not causing abnormal behavior to appear in normal mice received the administration. Then, following a 29-day washout period, a second administration was conducted. Upon the administration on the day 10 and the day 29, MAP was also administered to the SAL administrated group in the same amount as administered to the MAP administrated group.

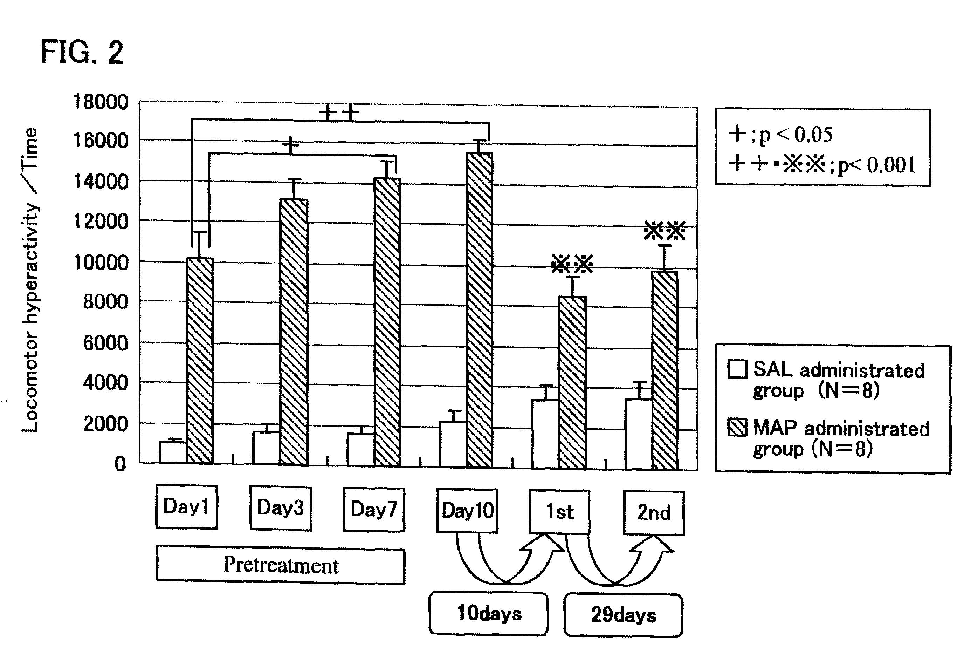

FIG. 2 shows the results. The horizontal axis shows date and the longitudinal axis shows locomotor hyperactivities per hour. During the pretreatment, activity quantity in an hour was measured after MAP or saline administration on Days 1, 3, 7, and 10. It is proven that activity quantity was increased in a time dependent manner as shown FIG. 2. In the case of a typical drug administration, tolerance is formed to gradually decrease influences of the administered drug. However, it was confirmed that the reverse phenomenon occurred so that reverse tolerance was formed.

In addition, comparing the MAP administrated group with the SAL administrated group, in both administrations on day 10 and day 29, it was clear that the activity quantity of the MAP administrated group was higher, so that it was confirmed that influences of the drug were maintained even after a longer than one-month washout period.

### Test to confirm effects of administration with fluoxetine

### Example 1

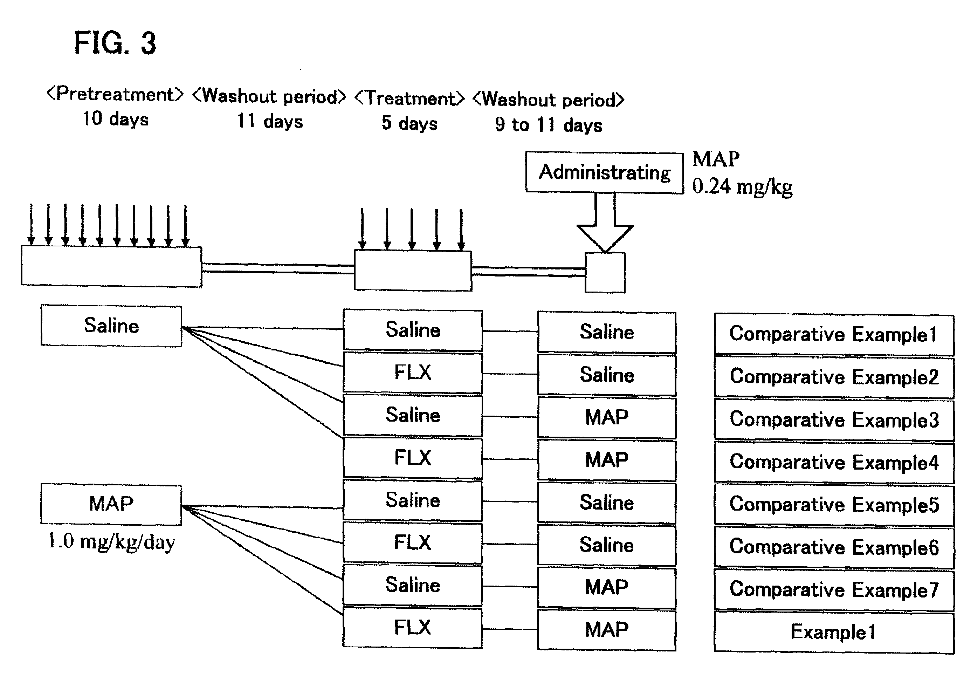

FIG. 3 shows the test procedure to confirm effects of administration with fluoxetine (hereinafter, may be also referred to as "FLX"). Regarding formation of reverse tolerance, 1 mg/kg/day MAP was administered to eight-week-old male ddy mice for ten days as a pretreatment in a similar way to the abovementioned method. After a 11-day washout period, 10 mg/kg/day fluoxetine was then administered. Fluoxetine was prepared by dissolving in saline and administered by subcutaneous injection. In addition, after 9 to 11-day washout period, 0.24 mg/kg MAP was administered, the locomotor hyperactivity of the mice was measured and evaluated one hour later. Comparative Examples 1 to 7

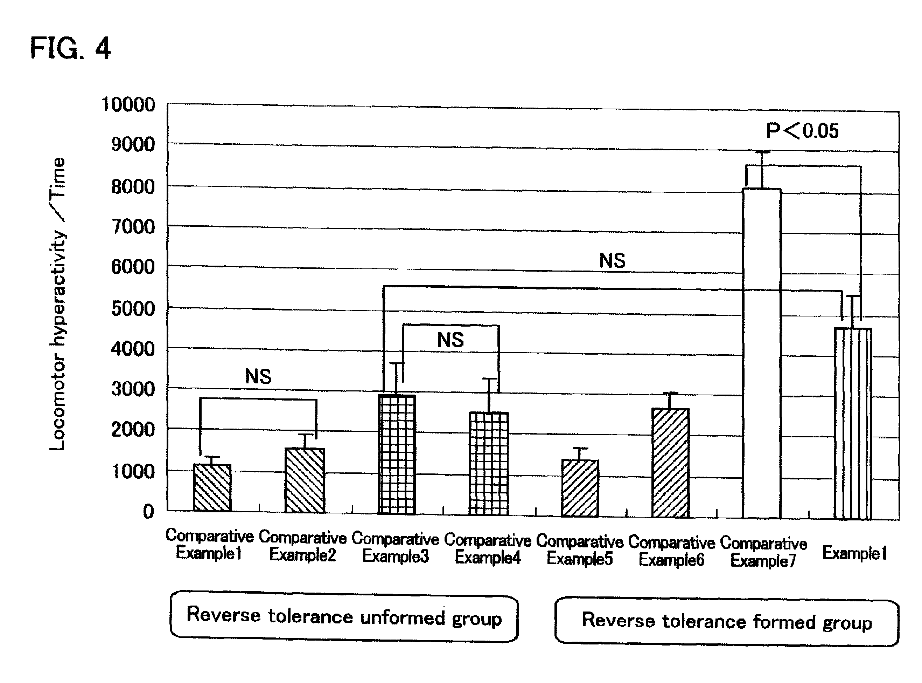

In addition, to observe influences and the like on administration with fluoxetine alone, the following tests were conducted in a similar way to the abovementioned test, as shown in FIG. 3: Comparative Examples 1-4 in which saline was used in the pretreatment, whereby reverse tolerance was not formed; Comparative Examples 5 and 7 in which after reverse tolerance was formed, saline was administered, whereby the reverse tolerance was maintained; and Comparative Example 6 in which after reverse tolerance was formed, fluoxetine was administered to allow the reverse tolerance to disappear, and then saline was administered. FIG. 4 shows the results.

Comparing Example 1 with Comparative Example 7 in which FLX was not administered in the treatment phase, it was confirmed that locomotor hyperactivity decreased by administering FLX. In addition, comparing Example 1 with Comparative Example 3 in which reverse tolerance was not formed, significant differences were not observed from the result, so that it was confirmed that reverse tolerance disappeared by administrating FLX. In addition, Comparing Comparative Example 1 with Comparative Example 2 and Comparative Example 3 with Comparative Example 4, there were no significant differences between the FLX administrated group and the FLX non-administrated group, so that it was confirmed that FLX itself did not decrease the activity quantity. Test to confirm effects of administration with paroxetine

### Example 1

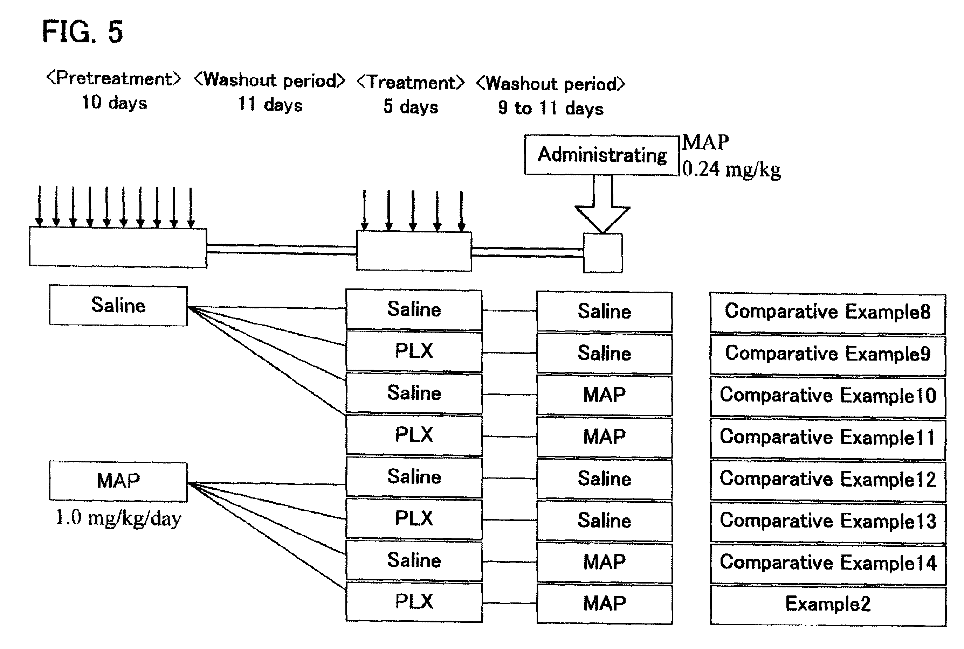

FIG. 5 shows the test procedure to confirm effects of administration with paroxetine (hereinafter, may be also referred to as "PLX"). Regarding formation of reverse tolerance, MAP 1 mg/kg/day paroxetine was administered to eight-week-old male ddy mice for ten days as a pretreatment in a similar way to the abovementioned method. After an 11-day washout period, 8 mg/kg/day paroxetine was then administered. Paroxetine was prepared by dissolving in saline and administered by subcutaneous injection. In addition, after 9 to 11-day washout period, 0.24 mg/kg MAP was administered, and the locomotor hyperactivity of the mice was measured and evaluated one hour later. Comparative Examples 8 to 14

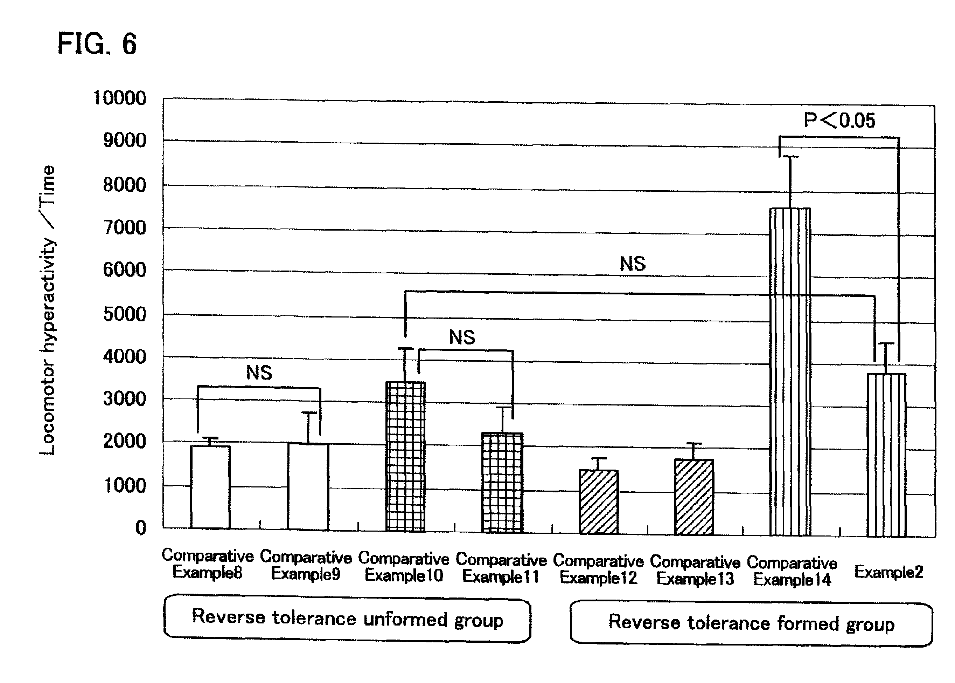

In addition, to observe influences and the like of administration with paroxetine alone, the following tests were conducted in a similar way to the abovementioned test, as shown in FIG. 5: Comparative Examples 8 to 11 in which saline was used in the pretreatment, whereby reverse tolerance was not formed; Comparative Examples 12 and 14 in which after reverse tolerance was formed, saline was administered, whereby the reverse tolerance was maintained; and Comparative Example 13 in which after reverse tolerance was formed, paroxetine was administered to allow the reverse tolerance to disappear, and then saline was administered. FIG. 6 shows the result.

Comparing Example 2 with Comparative Example 14 in which PLX was not administered in the treatment phase, it was confirmed that locomotor hyperactivity decreased by administering PLX. In addition, comparing Example 2 with Comparative Example 10 in which reverse tolerance was not formed, no significant differences were not observed from the result, so that it was confirmed that reverse tolerance disappeared by administrating PLX. In addition, Comparing Comparative Example 8 with Comparative Example 9 and Comparative Example 10 with Comparative Example 11, there were no significant differences between the PLX administrated group and the PLX non-administrated group, so that it was confirmed that PLX itself did not decrease activity quantity.

## Claims

1. A therapeutic agent for preventing recurrence of psychostimulant-induced psychosis and schizophrenia comprising a drug that amplifies synaptic serotonin signals as an active component.

2. A therapeutic agent for preventing recurrence of psychostimulant-induced psychosis and schizophrenia according to claim 1, wherein the drug that amplifies synaptic serotonin signals is a selective serotonin reuptake inhibitor (SSRI) or a double serotonin-noradrenaline reuptake inhibitor (SNRI).

3. The therapeutic agent for preventing recurrence of psychostimulant-induced psychosis and schizophrenia according to claim 1 or 2, wherein the drug that amplifies synaptic serotonin signals is fluoxetine (C₁₇H₁₈F₃NO) represented by the following formula (1), paroxetine (C₁₉H₂₀FNO₃) represented by the following formula (2), and/or derivatives thereof.
